(19) Europäisches Patentamt
European Patent Office
Office européen des brevets

(11) Numéro de publication: **0 435 737 B1**

(12) **FASCICULE DE BREVET EUROPEEN**

(45) Date de publication de fascicule du brevet: **19.07.95**

(51) Int. Cl.[6]: **C07C 15/16**, C07C 2/86, C07C 7/148

(21) Numéro de dépôt: **90403646.4**

(22) Date de dépôt: **18.12.90**

(54) **Procédés de synthèse de benzyltoluène et dibenzyltoluène à faible teneur en chlore.**

(30) Priorité: **28.12.89 FR 8917365**

(43) Date de publication de la demande:
**03.07.91 Bulletin 91/27**

(45) Mention de la délivrance du brevet:
**19.07.95 Bulletin 95/29**

(84) Etats contractants désignés:
**AT BE CH DE DK ES FR GB GR IT LI LU NL SE**

(56) Documents cités:
**EP-A- 0 225 849**
**EP-A- 0 250 748**
**EP-A- 0 306 398**
**GB-A- 2 009 224**

**CHEMICAL ABSTRACTS, vol. 112, no. 9, 26 février 1990, résumé no. 76576u,Columbus, Ohio, US; & SU-A-1 498 746 (E.V. VARSHAVER et al.) 07-08-1989**

(73) Titulaire: **ELF ATOCHEM S.A.**
**4 & 8, Cours Michelet**
**La Défense 10**
**F-92800 Puteaux (FR)**

(72) Inventeur: **Commandeur, Raymond**
**Le Rocher,**
**Avenue de Vénaria**
**F-38220 Vizille (FR)**
Inventeur: **Missos, Daniel**
**1, rue du 4ème Régiment de Génie**
**F-38000 Grenoble (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention concerne un procédé de synthèse de benzyltoluène et dibenzyltoluène à faible teneur en chlore. On a décrit dans le brevet européen EP 136 230 des compositions d'oligomères de polyarylalcanes utiles comme liquides diélectriques. Ces compositions sont essentiellement à base de benzyltoluène et dibenzyltoluène et sont préparées par chloration du toluène en chlorure de benzyle puis condensation du chlorure de benzyle sur le toluène restant par une réaction du type FRIEDEL et CRAFTS.

Dans ce procédé, comme dans tout procédé faisant intervenir une réaction de FRIEDEL et CRAFTS, il n'est pas toujours facile d'éliminer complètement l'acide chlorhydrique résultant de la condensation du chlorure de benzyle sur le toluène. De plus, le chlorure de benzyle peut aussi contenir du chlorure de chlorobenzyle $C_6H_4Cl\text{-}CH_2Cl$ qui par réaction FRIEDEL et CRAFTS avec le toluène donne du chlorobenzyltoluène $C_6H_4Cl\text{-}CH_2\text{-}C_6H_4\text{-}CH_3$.

Pour la plupart des applications diélectriques, on souhaite un produit ne contenant pas de chlore. Dans la demande de brevet EP 306 398, il est décrit un procédé de destruction de produits organiques halogénés sur les carbones aryliques, en particulier l'exemple 1 montre l'élimination du chlorobenzyltoluène contenu dans du dibenzyltoluène. Jusqu'à présent on avait toujours procédé dès la fin de la réaction de condensation FRIEDEL et CRAFTS à la destruction du catalyseur, par exemple par lavage à l'acide chlorhydrique dilué suivi d'un lavage à l'eau jusqu'à neutralité.

La demanderesse a découvert qu'en utilisant le chlorure ferrique comme catalyseur de FRIEDEL et CRAFTS, il n'est pas nécessaire d'effectuer un lavage pour l'éliminer et qu'on pouvait effectuer une déchloration sur le produit brut de condensation.

La présente invention est donc un procédé de synthèse d'oligomères de benzyltoluène dans lequel on effectue la condensation du chlorure de benzyle sur le toluène ou sur des oligomères inférieurs de benzyltoluène en présence de chlorure ferrique, puis, avant la récupération des oligomères ou met en contact le mélange réactionnel précédent, sous éliminer le chlorure ferrique, avec du sodium ou un alcoolate de métal alcalin pour éliminer les produits chlorés organiques.

La condensation du chlorure de benzyle sur le toluène est une réaction connue en soi qui peut s'effectuer en présence de tout catalyseur de FRIEDEL et CRAFTS et donc en particulier du chlorure ferrique. On appelle oligomères du benzyltoluène un mélange d'isomères de formule A :

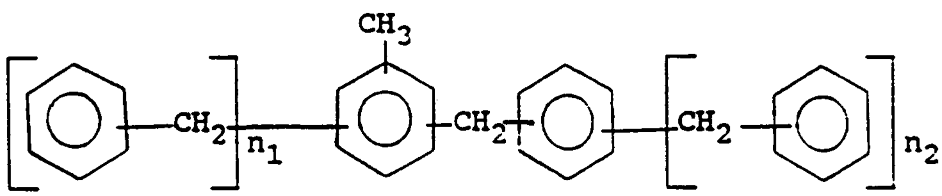

(A)

dans laquelle $n_1$ et $n_2$ valent 0,1 ou 2 et $n_1 + n_2$ est inférieur ou égal à 3.

On appelle benzyltoluène les oligomères dans lesquels $n_1 + n_2 = 0$ et dibenzyltoluène les oligomères dans lesquels $n_1 + n_2 = 1$.

Les oligomères inférieurs du benzyltoluène sont semblables aux oligomères du benzyltoluène mais contiennent moins de motifs benzyl. Par exemple le benzyltoluène ($n_1 + n_2 = 0$) est un oligomère inférieur du benzyltoluène par comparaison avec le dibenzyltoluène ($n_1 + n_2 = 1$). Si on condense par exemple du benzyltoluène ($n_1 + n_2 = 0$) avec du chlorure de benzyle on peut obtenir au moins du dibenzyltoluène (($n_1 + n_2 = 1$).

On ne sortirait pas du cadre de l'invention si la condensation du chlorure de benzyle s'effectue sur un mélange de toluène et d'oligomères inférieurs du dibenzyltoluène.

La condensation a lieu en pratique à une température comprise entre 50 et 150 °C. La quantité de chlorure ferrique est comprise habituellement entre 50 ppm et 1 % en poids de la masse réactionnelle.

Le mélange réactionnel qu'on vient d'obtenir et qui est constitué essentiellement d'oligomères de benzyltoluène, et d'un excès éventuel de toluène peut contenir des produits chlorés organiques tels que des chlorotoluènes, du chlorobenzyltoluène et d'une façon générale des oligomères de benzyltoluène portant un ou plusieurs atomes de chlore sur le noyau benzénique. Ces produits ont été apportés ou ont été formés à partir des impuretés du chlorure de benzyle ou par le chlorure ferrique.

Ledit mélange réactionnel est directement traité pour éliminer les produits chlorés organiques, c'est-à-dire sans procéder à l'élimination du catalyseur de condensation, le chlorure ferrique.

On peut utiliser tout procédé de destruction des produits chlorés organiques, par exemple le procédé décrit dans EP 306 398 utilisant un alcoolate de métal alcalin, le procédé décrit dans EP 225 849 utilisant le sodium métal et le procédé décrit dans EP 250 748 utilisant un alcoolate lourd, le contenu de ces demandes étant incorporé dans la présente invention. On préfère utiliser le procédé de déchloration décrit dans EP 306 398.

Selon le procédé préféré, on met en contact le mélange réactionnel obtenu en fin de condensation avec un alcoolate et on porte l'ensemble sous agitation à une température comprise entre 220 et 320 °C. Il est recommandé entre la fin de la condensation du chlorure de benzyle et la déchloration, d'éliminer par distillation le toluène excédentaire éventuellement présent dans le mélange réactionnel. Ce toluène peut être réutilisé en amont.

Après le traitement de déchloration, il suffit d'une simple distillation pour récupérer les oligomères de benzyltoluène à faible teneur en chlore. On obtient en pied une fraction lourde contenant les restes de l'agent déchlorant, du NaCl, des sels de fer et des oligomères lourds de benzyltoluène.

On ne sortirait pas du cadre de l'invention si on recyclait, en partie ou en totalité, cette fraction lourde obtenue en pied et qu'on l'utilisait seule ou en mélange avec le produit mis en oeuvre pour détruire les produits chlorés organiques.

On ne sortirait pas du cadre de l'invention si après la condensation et avant la déchloration on recyclait à l'étape de condensation en tout ou partie le benzyltoluène. On peut alors sortir du mélange réactionnel le toluène et le benzyltoluène, le toluène pouvant être recyclé en amont pour faire du chlorure de benzyle ou seulement recyclé à la condensation.

L'avantage de recycler en partie ou en totalité le benzyltoluène est d'augmenter la proportion de dibenzyltoluène dans les oligomères.

Le chlorure de benzyle peut contenir aussi du chlorure de benzylidène on obtient alors en mélange avec les oligomères du benzyltoluène, des oligomères du ditolylphénylméthane qui sont un mélange d'isomères de formule B:

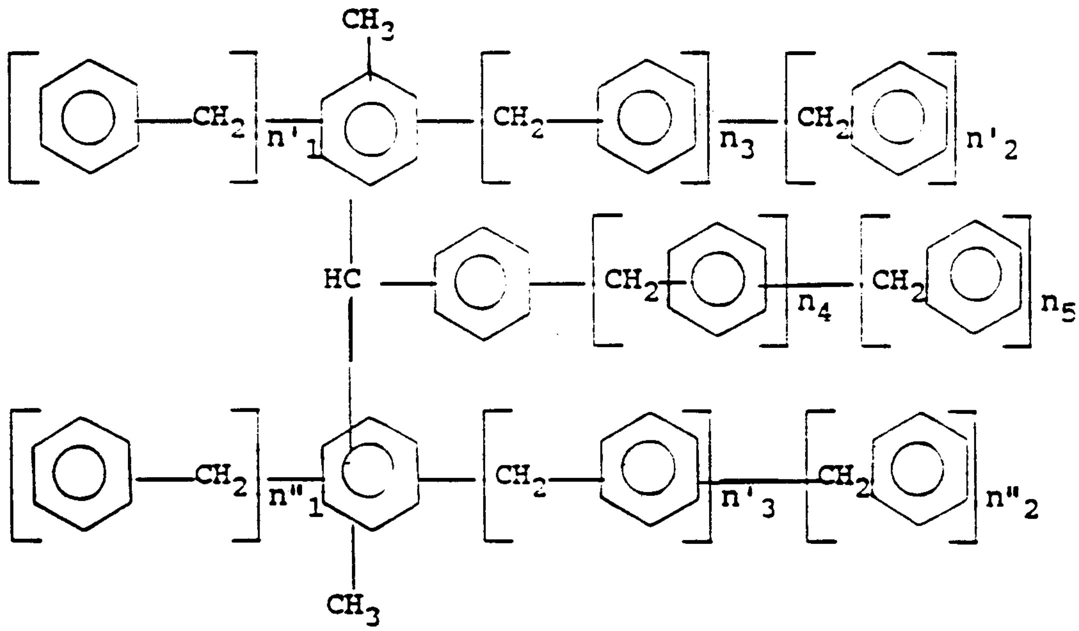

dans laquelle $n'_1$, $n''_1$ et $n_4$ valent 0,1 ou 2

$n'_2$, $n''_2$, $n_3$, $n'_3$ et $n_5$ valent 0 ou 1

sachant que $n'_1 + n''_1 + n'_2 + n''_2 + n_3 + n'_3 + n_4 + n_5$,

qu'on désigne par Sn est inférieur ou égal à 2.

On ne sortirait pas du cadre de l'invention si on effectuait la chloration du toluène puis qu'on procédait directement sur ce mélange à la condensation en présence de chlorure ferrique. Après cette chloration on peut éventuellement distiller pour séparer le chlorure de benzylidène.

Il va de soi que le procédé décrit peut être mis en oeuvre en continu ou en discontinu.

Ces oligomères sont utilisés comme fluides diélectriques.

Pour qu'ils puissent être utilisés comme diélectriques, il est recommandé de les purifier selon une technique qui consiste à employer une terre de foulon ou d'alumine activée, soit seules, soit en mélange, selon les techniques spécifiques connues dans le secteur des liquides diélectriques.

De même, il peut être avantageux d'ajouter des stabilisants du type époxyde ou d'autre nature comme par exemple le tétraphénylétain ou des composés anthraquinoniques employés en courant continu.

Ces adjuvants sont généralement des accepteurs d'acide chlorhydrique et sont ajoutés en quantités variables entre 0,001 et 10%, de préférence entre 0,01 et 0,3%.

## EXEMPLE 1

Dans un réacteur muni d'une agitation, d'un condenseur et d'un injecteur d'azote, on place 3 moles de toluène et 60 mg de chlorure ferrique. On porte l'ensemble à 100 ° C et on coule 1 mole de chlorure de benzyle en 1 heure. La masse est maintenue encore 1 heure à 100 ° C sous agitation. L'excès de toluène est éliminé par distillation sous vide. Le résidu composé d'oligomères de benzyltoluènes de type (A) est traité avec 1% de méthylate de sodium pendant 6 heures à 290 ° C sous couverture d'azote. Le mélange est ensuite soumis à une évaporation flash à 300 ° C sous 2 mm de mercure et conduit à un distillat constitué par un mélange d'oligomères de type (A) ayant la composition pondérale suivante:

$n_1 + n_2 = 0$ 66%

$n_1 + n_2 = 1$ 24%

$n_1 + n_2 = 2$ 10%

La teneur en chlore est inférieure à 5 ppm.

## EXEMPLE 2

On effectue un essai identique à celui décrit dans l'exemple 1 en utilisant le toluène non réagi de cette opération et en complétant avec du toluène frais.

Le mélange d'oligomères de type A obtenu après distillation a une teneur en chlore inférieure à 5 ppm.

## EXEMPLE 3

Dans un réacteur muni d'une agitation, d'un condenseur on charge 31,5 moles de benzyltoluène et 3,5 g de chlorure ferrique. On porte à 130 ° C et on coule 6,3 moles de chlorure de benzyle en 4 heures. Le milieu réactionnel est ensuite maintenu encore 1 heure à 130 ° C. Le milieu est ensuite traité pendant 3 heures à 290 ° C par 50 g de méthylate de sodium. La masse réactionnelle est ensuite soumise à une distillation sous vide de 0,5 mm de mercure avec colonne à garnissage emplie d'anneaux de verre (4 plateaux). La première fraction distillant vers 100 ° C contient le benzyltoluène non réagi (4000 g). La fraction suivante distillant entre 180 et 185 ° C (1000 g) a une pureté de 98% en dibenzyltoluène, c'est-à-dire en oligomère (A) dans lequel $n_1 + n_2 = 1$.

La teneur en chlore est inférieure à 5 ppm.

## EXEMPLE 4

Dans un réacteur muni d'une agitation, d'un condenseur, d'un tube d'alimentation de chlore et d'une lampe PHILIPS TLADK de 30 Watts, on place 4 moles de toluène. On introduit ensuite 1 mole de chlore gazeux en maintenant la température à 80 ° C durant 1 heure. Ce mélange est ensuite placé dans une ampoule de coulage et est introduit en 2 heures dans un réacteur muni d'une agitation et contenant 2 moles de toluène avec 100 mg de chlorure ferrique à 100 ° C. Le milieu réactionnel est maintenu encore 2 heures à 100 ° C avec agitation. L'excès de toluène est éliminé par distillation sous vide. Le résidu composé d'oligomères du benzyltoluène (A) et des oligomères dérivés du diolylphénylméthane (B) est traité avec 2% de méthylate de sodium pendant 6 heures à 290 ° C sous couverture d'azote.

Le mélange est ensuite soumis à une évaporation flash à 300 °C sous 2 mm de mercure et conduit à un distillat constitué d'un mélange des composés suivants:

| | | |
|---|---|---|
| **TYPE A**: | $n_1 + n_2 = 0$ | 78% |
| | $n_1 + n_2 = 1$ | 15,5% |
| | $n_1 + n_2 = 2$ | 4% |
| **TYPE B**: | Sn = 0 | 2% |
| | Sn = 1 | 0,5% |

La teneur en chlore est inférieure à 5 ppm.

## EXEMPLE 5

On effectue un essai identique à celui décrit dans l'exemple 4 en utilisant le toluène non réagi de cette opération et en complétant avec du toluène frais.

Le mélange d'oligomères de type (A) et (B) obtenu après distillation a une teneur en chlore inférieure à 5 ppm.

## EXEMPLE 6

On effectue un essai identique à celui décrit dans l'exemple 1. Le mélange d'oligomères brut obtenu après séparation du toluène non réagi est traité pendant 6 heures à 290 °C en présence du résidu de distillation de l'exemple 1 sous couverture d'azote. La masse est ensuite soumise à une distillation à 300 °C sous 2 mm de mercure. Le distillat est constitué par un mélange d'oligomères de type (A) ayant une teneur en chlore inférieure à 5 ppm.

**Revendications**

1. Procédé de synthèse d'oligomères de benzyltoluène dans lequel on effectue la condensation du chlorure de benzyle sur le toluène ou sur des oligomères inférieurs de benzyltoluène en présence de chlorure ferrique puis, avant la réculération des oligomères, on met en contact le mélange réactionnel précédent, sans éliminer le chlorure ferrique, avec du sodium ou un alcoolate de métal alcalin pour éliminer les produits chlorés organiques.

2. Procédé selon la revendication 1, caractérisé en ce qu'on élimine les produits chlorés organiques par réaction avec du méthylate de sodium.

3. Procédé selon la revendication 1, caractérisé en ce qu'on élimine le toluène excédentaire avant d'éliminer les produits chlorés organiques.

4. Procédé selon l'une des revendications 1 à 3, caractérisé en ce qu'on recycle en tout ou partie le benzyltoluène à l'étape de condensation, avant de procéder à l'élimination des produits organiques chlorés.

5. Procédé selon l'une des revendications 1 à 4, caractérisé en ce qu'on recycle, en partie ou en totalité, à l'étape d'élimination des produits chlorés organiques la fraction lourde qu'on obtient dans la distillation pour récupérer les oligomères de benzyltoluène à faible teneur en chlore.

**Claims**

1. Process for the synthesis of benzyltoluene oligomers in which the condensation of benzyl chloride with toluene or with lower benzyltoluene oligomers is carried out in the presence of ferric chloride and then, before the oligomers are recovered, the preceding reaction mixture is, without removal of the ferric chloride, placed in contact with sodium or an alkali metal alcoholate, in order to remove the chlorinated organic products.

2. Process according to Claim 1, characterised in that the chlorinated organic products are removed by reaction with sodium methylate.

3. Process according to Claim 1, characterised in that the excess toluene is removed before the chlorinated organic products are removed.

4. Process according to one of Claims 1 to 3, characterised in that all or part of the benzyltoluene is recycled to the condensation stage before the removal of the chlorinated organic products is undertaken.

5. Process according to one of Claims 1 to 4, characterised in that the heavy fraction which is obtained in the distillation for recovering the benzyltoluene oligomers of low chlorine content is recycled, partially or wholly, to the stage of removal of the chlorinated organic products.

**Patentansprüche**

1. Verfahren zur Synthese von Benzyltoluololigomeren, bei dem Benzylchlorid mit Toluol oder niederen Oligomeren von Benzyltoluol in Gegenwart von Eisen(III)-chlorid kondensiert wird und die vorstehende Reaktionsmischung anschließend vor der Wiedergewinnung der Oligomeren ohne Entfernung des Eisen(III)-chlorids mit Natrium oder einem Alkalimetallalkoholat kontaktiert wird, um die chlorierten organischen Produkte zu entfernen.

2. Verfahren nach Anspruch 1, dadurch gekennzeichnet, daß die chlorierten organischen Produkte durch Reaktion mit Natriummethylat entfernt werden.

3. Verfahren nach Anspruch 1, dadurch gekennzeichnet, daß überschüssiges Toluol vor der Entfernung der chlorierten organischen Produkte entfernt wird.

4. Verfahren nach einem der Ansprüche 1 bis 3, dadurch gekennzeichnet, daß Benzyltoluol im Kondensationsschritt vor der Entfernung der chlorierten organischen Produkte vollständig oder teilweise dein Kreislauf wieder zugeführt wird.

5. Verfahren nach einem der Ansprüche 1 bis 4, dadurch gekennzeichnet, daß während des Schrittes zur Entfernung der chlorierten organischen Produkte, die bei der Destillation zur Wiedergewinnung der Benzyltoluololigomere mit geringem Chlorgehalt gewonnene schwere Fraktion teilweise oder vollständig dein Kreislauf wieder zugeführt wird.